# EUROPEAN PATENT APPLICATION

(11) **EP 2 339 337 A2**
(43) Date of publication of application: **29.06.2011**
(21) Application number: 10015775.9
(22) Date of filing: 17.12.2010
(51) Int. Cl.: G01N 27/327

(54) **System for reading analyte test elements and for other uses**

(30) Priority: 23.12.2009 US 645520
(71) Applicant: Roche Diagnostics GmbH, 68305 Mannheim (DE); F.Hoffmann-La Roche AG, 4070 Basel (CH)
(72) Inventor: Wooldridge, Scott, Carmel IN 46033 (US)

(57) **Abstract**

A multiuse meter (300) is operable to communicate with an analyte test element (110). The meter (300) has access to memory locations (122) of an inserted memory unit (120).

## Description

### BACKGROUND

There are significant and increasing needs to measure analytes present in a fluid sample. For example, patients with diabetes can benefit from measurement of their blood glucose. Those potentially at risk of heart disease can benefit from measurement of cholesterols and triglycerides among other analytes. These are but a few examples of patient benefit from analyte measurement in biological samples. Advancements in the medical sciences are identifying a growing number of analytes including molecules, lipids, carbohydrates, amino acids, antibodies, proteins, nucleic acids, peptides, viruses, bacteria, markers, drugs, toxins, and other analytes which could be measured to identify a number of diseases, disorders and conditions. Present approaches to measuring analytes in a biological sample are subject to a number of drawbacks, limitations, disadvantages and problems. There is a need for the unique, beneficial and inventive solutions disclosed herein.

### SUMMARY

One embodiment includes a memory unit for use in connection with a plurality of fluid sample test elements, wherein the memory unit comprises a plurality of memory portions. In one aspect of the embodiment, the memory unit comprises at least one memory portion configured to communicate calibration and expiration information relating to a lot of test elements, to a meter operably connectable with such test elements, and at least one other memory portion configured for storage and communication of data, such as measurement results, relating to the use of the test elements in analyzing a fluid sample. In another aspect of the embodiment, the memory unit comprises an encrypted memory portion and a password protected memory portion. In a further aspect of the embodiment, the memory unit comprises a further memory portion configured for enabling the meter for uses not relating to the analysis of fluid samples applied to the test elements, such as access to instructional information, music files, games, and other audio/visual uses of hand held electronic devices, all of which may be stored in this further memory portion.

Further embodiments include apparatuses, systems, methods, kits and combinations of test elements and memory units.

### BRIEF DESCRIPTION OF THE FIGURES

Fig. 1 is a schematic diagram of an exemplary memory unit and exemplary test elements.
Fig. 2 is a is a schematic diagram of an exemplary memory unit.
Fig. 3 is a schematic diagram of an exemplary memory unit and an exemplary test element operatively connected with an exemplary meter.
Fig. 4 is a schematic diagram of an exemplary memory unit and an exemplary test element operatively connected with an exemplary meter.
Fig. 5 is a flow diagram of an exemplary method.

### DETAILED DESCRIPTION

For the purposes of clearly, concisely and exactly describing exemplary embodiments of the invention, the manner and process of making and using the same, and to enable the practice, making and use of the same, reference will now be made to the exemplary embodiments illustrated in the figures and specific language will be used to describe the same. It shall nevertheless be understood that no limitation of the scope of the invention is thereby created, and that the invention includes and protects such alterations and modifications to the exemplary embodiments, and such further applications of the exemplary embodiments as would occur to one skilled in the art to which the invention relates.

### Test Element Systems

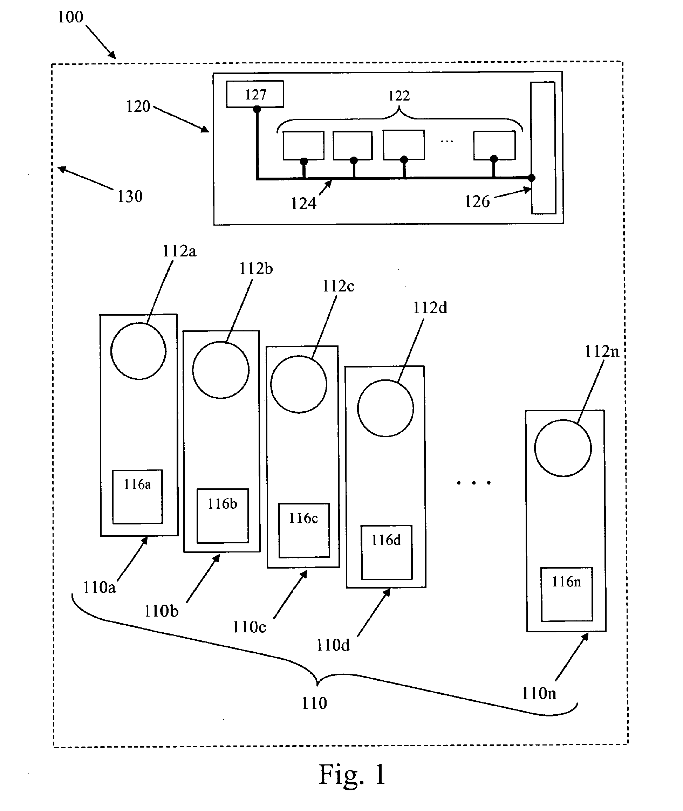

Fig. 1 illustrates a system 100 which includes a set of test elements 110 configured for analysis of a fluid sample. Test elements 110a through 110n are depicted to illustrate that various embodiments may include as few as one test element and as many as dozens, hundreds, or greater numbers of test elements. For example, some embodiments include batches of 100, 150, 200 or other numbers of test elements. In some embodiments, test elements 110 are provided as disposable test strips. In other embodiments, test elements 110 are provided as non-strip test elements. In some embodiments, test elements 110 are provided as reusable test elements. In further embodiments test elements 110 are provided in other forms including, for example, micro-arrays, lab-on-chip devices, bio-chips, bio-discs, or bio-CDs.

Test elements 110a through 110n include respective test regions 112a through 112n which are operable to receive samples of fluid and provide one or more indicia relating to one or more analytes in the sample. In certain embodiments test regions 112a through 112n are electrochemical test regions which are operable to receive respective samples of fluid, react their respective samples of fluid with one or more reagents, and provide one or more indicia relating to one or more analytes in the sample. One embodiment of an analytical test element comprising an electrochemical test element is the ACCU-CHEKⓇ Aviva test strip, which is described in U.S. Patent Application Publication No. 2005/0016844, the disclosure of which is incorporated by reference. Other embodiments include optical test elements, for example, the ACCU-CHEKⓇ Compact test strip, which is described in U.S. Patent No. 7,008,799, the disclosure of which is incorporated by reference. These and other test elements are distributed in the United States by Roche Diagnostics Corporation of Indianapolis, Indiana.

There are multiple embodiments which include a variety of sets of test elements. Some embodiments include sets of test elements having respective test regions operable to provide indicia of the same analyte or the same analytes. Some embodiments include test element sets having respective test regions operable to provide indicia of various different analytes. Some embodiments, for example the aforementioned ACCU-CHECKⓇ Aviva and Compact test strips, comprise biological test elements operable to provide indicia relating to blood glucose. Other embodiments include biological test elements operable to provide indicia of cholesterols, triglycerides and/or other analytes associated with cardiovascular health. Other embodiments include biological test elements operable to provide indicia of a variety of additional or alternate health-related sample analyte molecules, such as lipids, carbohydrates, amino acids, antibodies, proteins, nucleic acids, peptides, viruses, bacteria, markers, drugs, toxins, and other biological analytes which can be measured to identify a number of diseases, disorders and conditions. Other embodiments include biological test elements operable to provide indicia of combinations of the foregoing and/or other analytes.

Typical test elements 1 10a through 110n include interfaces 116a through 116n, respectively. Interfaces 116a through 116n are operable to provide power and/or test signal information from one or more external sources to test elements 110a through 110n, respectively. In some embodiments interfaces 116a through 116n are operable to couple test elements 110a through 110n with a meter or other testing device and allow power and/or testing information to be communicated therebetween. Interfaces 116a through 116n may include electrically conductive interfaces, wireless interfaces, optical interfaces or other interfaces operable to provide one or more power and/or communication links between test elements 110 and a meter or other device(s).

In certain exemplary embodiments, test elements 110a through 110n are operable to provide an electrical potential to one or more electrodes of test regions 112a through 112n, and provide information of a response to the electrical potential to interfaces 116a through 116n. In some embodiments the response includes information indicative of a current response such as a Cottrell current response. Interfaces 116a through 116n are operable to provide information of a response to the electrical potential to a meter or another test device. In some embodiments the information is indicative of the concentration of an analyte in a sample. In some embodiments, the information is indicative of the quantity of an analyte in a sample. In some embodiments, the information is qualitative information of an analyte in a sample. Some embodiments include a meter which is operable to detect the presence or absence of a sample, evaluate sample dose sufficiency, time sample incubation, and time sample measurement via communications with interfaces such as interfaces 116a through 116n.

### Memory Units

As shown, system 100 also includes a memory unit 120. Memory unit 120 may be provided in combination with test elements 110, for example, in common packaging 130. In a typical test element system, a memory unit 120 is provided with a memory that stores calibration information and/or other information that pertains to a particular production lot of test elements. This test element information is communicated from the memory unit 120 to a meter 300 configured for use with the test elements. An example of a memory unit in this regard, which may also include information relating to measurement parameters, details of measurement step actions to be performed by the meter, etc., is disclosed in USP 5,366,609, the disclosure of which is hereby incorporated herein by reference. In that embodiment, the memory unit is provided on a pluggable key configured to interface in a corresponding receiving slot on a meter.

As will be appreciated from the present disclosure, the memory unit 120 of the present invention need not be provided specifically with a group of test elements 110. In other embodiments memory unit 120 may be provided separately from test elements 110. In some embodiments, memory unit 120 may be associated with test elements 110 at a point of sale. In some embodiments memory unit 120 may be updated by a user or operator by receiving a download or other transmission based upon information associated with test elements 110 such as a serial number, keycode, barcode, universal product code ("UPC") or other information.

In accordance with the present invention, memory unit 120 includes a number of memory locations 122 and interface 126 which are operatively coupled with communication link 124 which can be a bus or other type of communication link. In embodiments according the present invention, memory unit 120 may include a number of types of memory. Some embodiments include nonvolatile memory, for example, Flash, EEPROM, EPROM, and ROM. Some embodiments include volatile memory such as RAM or other types of volatile memory. Some embodiments include variations or combinations of the aforementioned memory types and/or other types of memory.

Memory unit 120 further includes control circuitry 127 which may be a microprocessor, microcontroller, ASIC, or other circuitry operable to control one or more functionalities of memory unit 120. In some embodiments control circuitry may include its own memory, or may utilize one or more of memory locations 122, or both. Some embodiments may omit control circuitry 127 while providing memory locations 122.

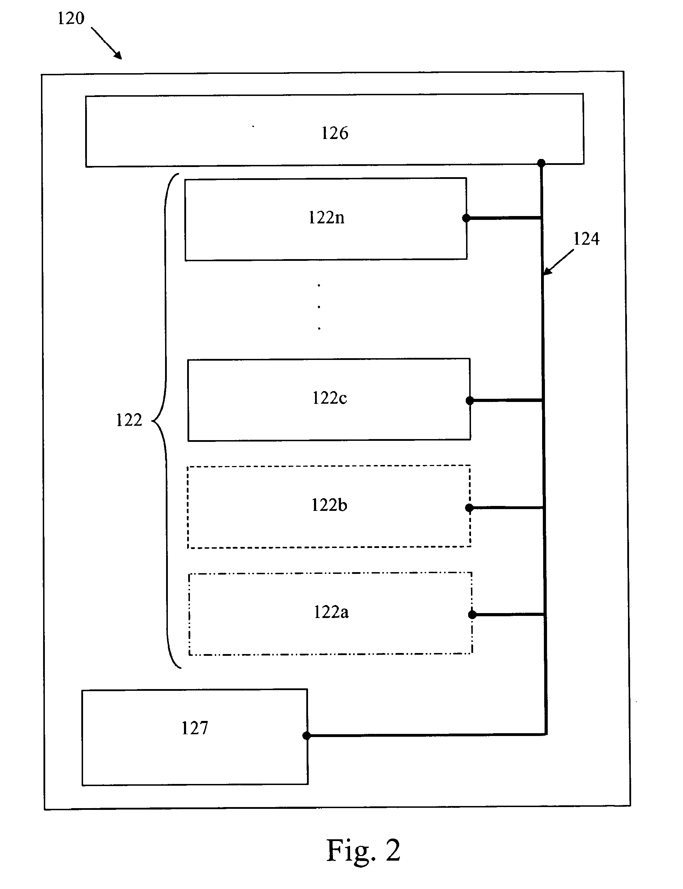

Fig. 2 illustrates memory unit 120 in further detail. Memory unit 120 includes a plurality of memory locations 122. As used herein the term memory locations includes sets of one or more memory locations and typically includes sets of multiple memory locations. The illustrated embodiment depicts memory locations 122a through 122n to illustrate that various embodiments may include various numbers of memory locations. In some embodiments, memory locations 122a through 122n may be provided as partitions of one or more common memory elements, for example, physical partitions, logical partitions, fixed partitions, dynamic partitions or combinations of those or other types of partitions. In some embodiments, memory locations 122a through 122n are provided in a single unitary memory. In some embodiments, memory locations 122a through 122n are provided in two or more discrete memories.

As was described above, memory unit 120 comprises at least one memory location 122 configured to store and communicate to a meter 300 with which it is operatively connected the calibration and/or other lot specific information relating to a particular lot of test elements. This information can be stored on the memory unit 120 as provided with a corresponding group of test elements 110, or the memory unit 120 may be obtained separately from the group of test elements. In one embodiment, appropriate means are provided for verifying the lot identification of a group of test elements 110 to which a separately obtained memory unit 120 corresponds. In other embodiments, however, a memory unit 120 already in the possession of a user can be uploaded via computer networking means, for example, with this information for a separately obtained group of test elements 110. Means for interfacing a memory unit 120 with a computer networking means, for example, will be appreciated by those of ordinary skill in the art and need not be further elaborated upon here.

In addition to the memory location for storing test element specific information, the memory unit 120 of the present invention further comprises one or more memory locations 122 relating to other uses of the memory unit. In one embodiment, one or more memory locations are provided for data storage outside of the meter corresponding to the test elements. Meters 300 for use with test elements 110 are typically provided with on-board memory storage capacity relating to, e.g., measurement results. For example, the ACCU-CHEKⓇ Aviva blood glucose meter is configured to store up to 500 results values. According to this embodiment of the present invention, however, the results values can be stored on the memory unit 120 in an identified memory location 122. This accomplishes at least two benefits. First, a meter 300 will not be required to comprise memory storage capacity, or at least not at the same capacity level. Second, the memory unit 120 will become portable for use with other meters while maintaining a comprehensive set of data values relating to measurement results.

In other embodiments, the at least one memory location 122 designated for data storage comprises a memory that can upload data to a meter and can download data from a meter. In yet other embodiments, the memory unit 120 can be interfaced with, e.g., the electronic records of a health care provider to upload analytical results relating to the user's measurements of, e.g., blood glucose levels from one visit to another to such health care provider's office. In yet other embodiments, the level of memory so designated is at least 1 gigabyte, and in other embodiments is at least 16 gigabytes. It will be appreciated by those of ordinary skill in the art, however, that the level of memory in a memory location is only limited by state of the art, and thus is contemplated to continue to grow. As such, the scope of this disclosure in this regard is not limited to the level of memory available at the time of filing this application.

In yet other embodiments, the memory unit 120 of the present invention further comprises one or more memory locations 122 relating to other uses of meter 300 that are generally or typically unrelated to the analytical use of the meter with the corresponding test elements 110. In one aspect, at least one memory location 122 is provided for storage of one or more of music files, video files, gaming files, and other entertainment related files that can be accessed and executed by a meter 300 that is equipped with corresponding capabilities. In another aspect, the memory unit 120 comprises at least one memory location 122 comprising files, codes, or other programming means that are uploadable or executable by the meter 300 to enable the meter with the functionality relating to the use of the entertainment related files. Other files that may be stored and accessed by the meter include documents, such as e-book files or even instructional information relating to the use of the meter.

As may be now more fully appreciated in view of the foregoing description, the embodiments of memory unit 120 of the present invention are useful for the conventional purposes of conveying test element specific information to a corresponding meter, but also for the purposes of one or both of expanded memory capabilities as well as less conventional uses of analytical meters. Indeed, the embodiments of the present invention contemplate a system of various analytical meters and test elements, e.g. blood glucose meter systems and cholesterol meter systems, which make use of a common memory unit.

Control circuitry 127 is operable to perform a number of functions relating to memory unit 120. Control circuitry 127 is operable to control input/output functions, encryption/decryption functions, password functions, identify verification functions, memory addressing functions, and other functions. Control circuitry 127 may further comprise functional programming relating to the various possible uses of a meter 300 in connection with the memory unit 120 of the present invention. Nevertheless, it is contemplated that some embodiments may omit control circuitry 127 while providing one or more memory locations and an input/output interface.

### Example 1

In one example of the use of such a system, a blood glucose meter and a cholesterol meter are provided, as well as respectively corresponding groups of glucose test elements and cholesterol test elements, each for example provided in a vial of 50 test elements. A memory unit is provided with each vial, and each is operatively connectable with each meter, and is configured with the respective test element specific information as well as analyte measurement specific information for both analyte meters. A user decides to utilize the memory unit from the blood glucose test element vial and inserts the memory unit into a corresponding slot on the blood glucose meter. The user performs several measurements over the course of several days. The user's cholesterol testing therapy regimen only requires a single analysis every few days. The user logs on to a website in order to download onto the blood glucose memory unit the test element specific information relating to the particular lot of cholesterol test elements, which information is stored in a separately accessible memory location on the memory unit. The memory unit now has the test element specific information for both vials of test elements, and was already configured with both glucose and cholesterol measurement specific information. The user is thus now able to use the memory unit with the cholesterol meter in order to conduct a cholesterol analysis using the cholesterol test elements, and can then store the cholesterol measurement results on the expanded storage memory location of the memory unit. In one embodiment, the blood glucose measurement data and cholesterol measurement data are correlated by time and date of the measurements for subsequent use by, for example, a health analysis software program that can download the results from the memory unit for analysis.

In addition to the foregoing, the user is able to store music files from her mp3 collection onto the memory unit. If the glucose meter and/or the cholesterol meter are equipped with music file playing capabilities, she will be able to also use either meter as a music player. Alternatively, the memory unit comprises a memory location or control circuitry that includes ROM programming that is accessible by both meters for use in enabling the user to play the music files through the meters.

### Example 2

In another example of the use of such a system, a diabetic patient owns more than one blood glucose meter, for example one for use at home and one for use at work. The memory unit provided with a vial of glucose test elements comprises memory locations for storing lot specific information for the vial of test elements as well as memory locations for storage of and access to measurement result data. The user is thus able to compile comprehensive data in a single location that relates to measurement analyses performed on two different meters.

### Example 3

In yet another example of the use of such a system, a universal meter is provided. A memory unit that has been obtained by a user for use with the universal meter is uploaded with information relating to (i) analyte measurement specific information for a blood glucose measurement using certain blood glucose test elements, (ii) test element specific information relating to a particular lot of the certain blood glucose test elements, (iii) analyte measurement specific information for a cholesterol measurement using certain cholesterol test elements, and (iv) test element specific information relating to a particular lot of the certain cholesterol test elements. The universal meter further comprises general music file playing capabilities, document display capabilities, as well as video file playing capabilities. A state of the art video and textual display is provided with the universal meter. The universal meter is further configured to operatively connect to both the certain blood glucose test elements and the certain cholesterol test elements. Each of the certain blood glucose test elements and the certain cholesterol test elements are configured with means discernible by the universal meter for distinguishing which type of test element is operatively connected to the meter. Such means are known in the current state of the art and further elaboration is not required for purposes of this disclosure.

A user operatively connects the memory unit with the universal meter. As a result, the user is able to conduct both blood glucose measurements and cholesterol measurements, and store the respective results data. The memory unit may be configured to segregate the results data or to combine them. The memory unit may be further configured with a control circuit programming that analyzes the results of each separately or combined, depending on the analysis programming provided.

The universal meter is relatively inexpensive because it has limited memory requirements as well as limited circuitry requirements as a result of the memory and substantive functional programming being accessed from the memory unit. The only significant functional requirements of the universal meter relate to the optional inclusion of entertainment capabilities, which as previously explained may also be provided on and accessed from the memory unit.

Memory locations 122a through 122n may be provided with a variety of security features including encryption, hidden memory locations, password protection, digital rights management ("DRM") and combinations of these and other security features. In some embodiments, information stored in one or more of memory locations 122a through 122n is encrypted so that only a user or device in possession of a key may decrypt information stored in an encrypted memory location. A variety of encryption systems and techniques may be used including, for example, block ciphers, stream ciphers, cryptographic hash functions, message authentication codes ("MACs"), digital signatures, Data Encryption Standard ("DES") encryption, triple-DES encryption, Advanced Encryption Standard ("AES") encryption, public-key encryption, Diffie-Hellman encryption, RSA encryption, elliptic curve encryption, PGP encryption, El Gamal encryption or combinations of these and/or other encryption systems and techniques.

Some embodiments utilize information-level encryption. In one such embodiment, one or more of memory locations 122 is imaged with or otherwise configured to store encrypted information. Only a user or device in possession of a key can meaningfully interpret the encrypted information. Some embodiments utilize software-level encryption in which software stored in a memory such as one or more of memory locations 122 or other memory locations is operable to provide encryption or decryption of input or output to or from one or more of memory locations 1.22. Some embodiments utilize hardware-level encryption in which hardware such as control circuitry 127 or a portion thereof carry out encryption or decryption of input or output to or from one or more of memory locations 122. Other embodiments use combinations of the aforementioned and/or other encryption systems and techniques.

In some embodiments, one or more of memory locations 122 is hidden. Hidden memory locations may be provided in a number of forms. For example, hidden memory locations may be visible only to a user or device possessing certain credentials such as a digital signature, a hash credential or other types of credentials. Hidden memory locations may be provided so that they are not a visible part of the directory structure seen by a user or device accessing memory unit 120. Hidden memory locations may be addressable exclusively by hardware, software or a combination thereof found within memory unit 120 and not addressable by an external user or device. In some such embodiments, information stored in one or more hidden memory locations may be provided to an external user or device via interface 126 based upon a request and credential verification process in which control circuitry 127 receives a request for information in a hidden memory location, verifies credentials of the requestor, and provides (or declines to provide) information in a hidden memory location to the requestor via interface 126.

In some embodiments control circuitry may require a key to be provided by the requestor which is used to decrypt the information provided to the requestor via interface 126. In some embodiments encrypted information is provided to the requestor via interface 126 and the requestor may perform decryption external to memory device 120 based upon a key. In some embodiments, communication between memory device 120 and a requestor may itself be encrypted so that input and/or output therebetween requires a key to meaningfully interpret. In some embodiments, one or more of memory locations 122 may be hidden and encrypted.

In some embodiments, one or more of memory locations 122 is password protected so that a password is required to access information stored therein. Some embodiments may include multiple memory locations with separate passwords. In some embodiments, one or more of memory locations 122 includes DRM-protected content for which access, duplication and transfer are restricted or controlled. Some embodiments include one or more encrypted memory locations and one or more password protected set of memory locations. Some embodiments include one or more encrypted memory locations, one or more password protected memory locations, and one or more memory locations storing information subject to DRM. Further embodiments include additional or alternate memory locations.

In the embodiment illustrated in Fig. 2, information in memory locations 122a is encrypted and hidden, information in memory locations 122b is password protected, and information memory locations 122c is subject to DRM controls. As indicated by the ellipsis and memory locations 122n, memory device 120 may include additional sets of memory locations having the foregoing attributes, other attributes or combinations thereof.

Hidden encrypted memory locations 122a store calibration information which relates to test elements 110 and can be used to calibrate a meter or other measurement device to test elements 110. Many test elements, for example blood glucose test elements, require that a measurement device such as a meter be calibrated in order to provide reliable and accurate measurements. Hidden encrypted memory locations 122a are also configured to store executable code or software which can be provided to a meter or other measurement device. The executable code or software may include updated or changed analyte analysis or measurement algorithms, operating system updates or changes, additional algorithms to provide additional analyte analysis or measurement capabilities, and/or other executable code or software.

Password protected memory locations 122b are configured to store password protected analyte measurement or analysis result information provided by a meter or other measurement device and/or other information desired to be password protected. Password protected information requires a user or device to provide a password in order to be granted access. The password may be a number such as a pin, a combination of letters, an alphanumeric combination, output of a biometric identifier such as a finger recognition, or combinations of these and/or other types of passwords. Password protected memory locations 122b may be sub-divided into multiple sub-locations each having a separate password. Multiple separate sets of memory locations each having a separate password can also be provided.

Memory locations 122c may be configured to store a variety of information. For example, memory locations 122c may be configured to store digital media content. Digital media content includes audio content, image content, video content, gaming content, or combinations thereof. Digital media content may be protected by DRM measures to ensure that it is not accessed, copied and/or transferred without authorization.

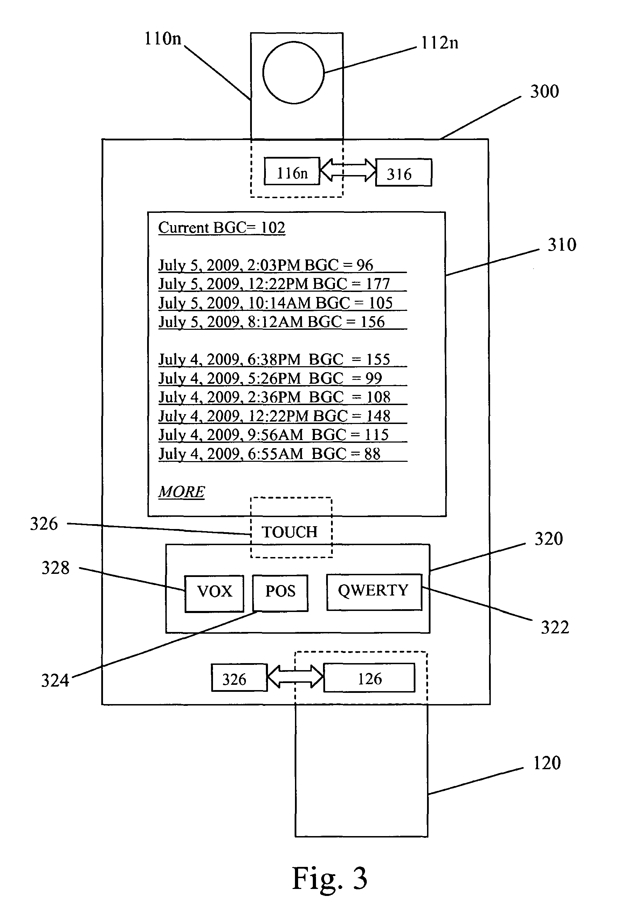

Fig. 3 illustrates a meter 300 operatively connected with test element 110n and memory unit 120. Interface 116 of test element 110n operatively connected with interface 316 of meter 300. Communication between interface 116 and interface 316 may be provided by an electrical interconnection, optical interconnection, wireless interconnection or combination of these and other types of communication links. Interface 126 of memory unit 120 is operatively connected with interface 326 of meter 300. Communication between interface 126 and interface 326 may be any of the types described in connection with interface 116 and interface 316.

Meter 300 includes a display 310 and user input 320. User input 320 may be of a number of forms. In some embodiments user input 320 includes a QWERTY keyboard interface 322 or other types of key interfaces such as numeric keypads and other types of character key inputs and interfaces. In some embodiments user input 320 includes positional input 324, for example, a five position input such as an up-down-left-right-center input. In some embodiments user input 320 includes a touch screen input 326 which is operable to receive input via display 310. Some embodiments include a voice-activated input 328. Some embodiments include combinations of these and/or other types of inputs. Meter 300 also includes processing or control circuitry and may also include audio outputs, additional input/output or communication interfaces, interfaces for additional test elements, interfaces for additional memory units, wireless telecommunication, voice and/or data capability, or combinations of these or other additional features. Additional features of exemplary meters and related electrical and optical components and their respective measurement techniques are described in U.S. Patent Nos. 5,352,351; 4,999,482; 5,438,271; 6,645,368; 5,997,817; 6,662,439; RE 36,268; 5,463,467; 5,424,035; 6,055,060; 6,906,802; and 5,889,585; the disclosures of which are hereby incorporated by reference.

As illustrated in Fig. 3, meter 300 has performed multiple analyses of samples provided by a user to test elements. Meter 300 has obtained calibration information relating to the test elements from memory unit 120 and used this information to analyze a sample provided to test elements. In the illustrated embodiment, meter 300 has measured glucose concentration of a sample of blood provided to test element 110n and is displaying the results of this measurement on display 310 as "Current BGC". Meter 300 has also previously analyzed multiple blood glucose levels of samples provided to other test elements and is displaying the dates, times and measurement values for these analyses on display 310. Meter 300 has stored the measurement information in a password protected memory location of memory unit 120. A password must be provided in order to access the stored measurement information. Meter 310 has also obtained executable code or software from memory 120, including updated or changed analyte measurement algorithms, updated or changed operating system information, algorithms to provide additional analyte measurement capabilities, and has used this additional executable code or software in measuring analyte characteristics and displaying and storing the results thereof.

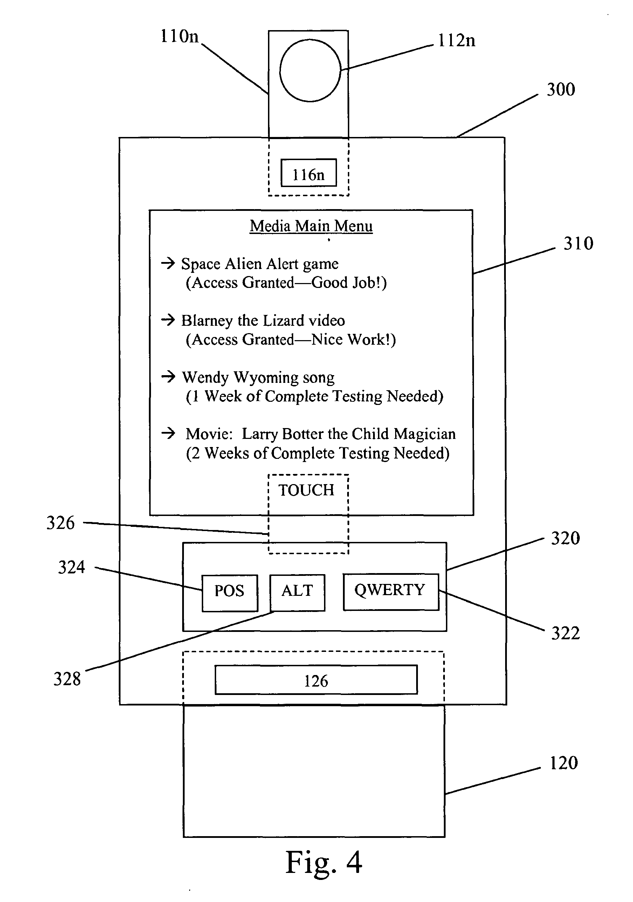

Fig. 4 illustrates meter 300, test element 110n and memory unit 120 in a configuration similar to that of Fig. 3. In Fig. 4, meter 300 is running an interactive media access management program in which a user is provided access to certain media content, and notified of additional requirements needed to gain access to additional media content. In the illustrated example, the user has been provided with access to a Space Alien Alert Game, and a Blarney the Lizard video, but must complete one additional week of testing to gain access to a song by Wendy Wyoming and a Larry Blotter movie. This media content has been provided to meter 300 from memory unit 120 and/or other similar memory units placed in operative communication with meter 300.

Media content may be provided to memory unit 120 and meter 300 in a number of manners. Media content may be provided with memory unit 120 or loaded onto memory unit 120 by a user. Media content may be stored in any memory location of memory unit 120 including encrypted memory, password protected memory, or other memory. Media content may reside in memory unit 120 and be accessed by meter 300 or transferred to meter 300 and accessed thereafter. Media content may also be provided with DRM controls to prevent unauthorized access, copying or transfer.

The interactive media access management program running on meter 300 includes a number of control features. For example, the number of stored analysis result entries required to gain access to any particular media may be set by an administrator, for example, a parent or guardian. The messages displayed by the interactive media access management program may also be customized or personalized by a user or administrator. A user may control interactive media access management program with user input 320 or another input. A user may select the media content she or he wishes to access and may select the output mode, for example, video and audio may be displayed in the display 310 and audio output of meter 300 or may be sent to one or more external outputs.

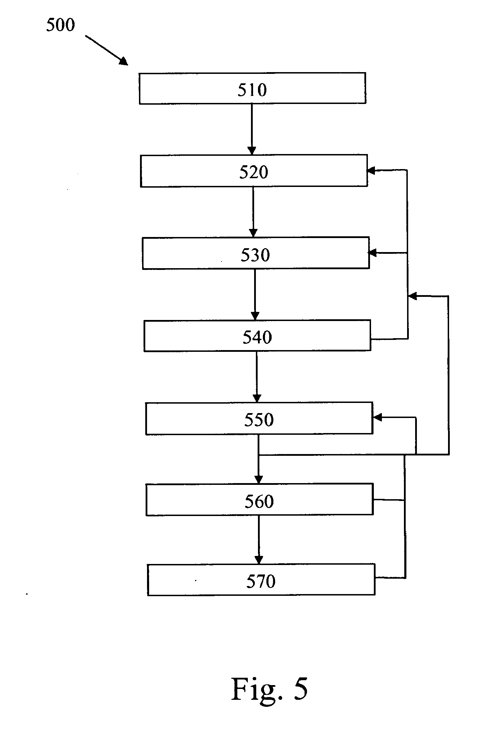

Fig. 5 illustrates a flow diagram describing a process 500. At operation 510 a system including a test element, a memory unit, and a meter is provided. The memory unit includes encrypted memory locations storing calibration information associated with the test element and executable instructions such as commuter code or software for a testing algorithm, password protected memory locations operable to receive and password protect measurement information, and additional memory locations including media content.

From operation 510 process 500 proceeds to operation 520 where the memory unit is placed in operative communication with the meter, the meter accesses the calibration information associated with the test element and executable instructions, and the meter configures itself to operate in accordance with the calibration information and executable instructions provided from the encrypted memory locations.

From operation 520 process 500 proceeds to operation 530 where a sample is provided to a test element, the test element is placed in operative communication with the meter, and the meter analyzes the sample in accordance with the calibration information and executable instructions provided from the encrypted memory locations.

From operation 530 process 500 proceeds to operation 540 where the results of the analysis from operation 520 are displayed and/or stored in a password protected memory location of the memory unit. From operation 540 process 500 may return to operation 520 or to operation 530 and operations 520, 530 and 540 may be repeated one or more times.

From operation 540 process 500 may proceed to operation 550, for example, based upon input from a user. At operation 550 a user may be prompted for a password in order to gain access to measurement information stored in a password protected memory location of the memory unit. This may occur while the memory unit is in operative communication with the meter, or after the memory unit has been placed in operative communication with another device. Password prompting may continue until a valid password is received. If a valid password is received process 500 proceeds to operation 560 where measurement information is made accessible to the user and may be displayed, downloaded or otherwise accessed.

From operation 550 process 500 may also return to operation 520 or to operation 530, and operations 520, 530, 540 and 550 may be repeated multiple times. Process 550 may also proceed to operation 560 where measurement information is processed and to operation 570 where output based upon the processing may be displayed. For example, a graphical illustration of actual testing events relative to target testing events may be displayed. Measurement averages, testing frequencies and various other types of output relating to testing and measurement may be displayed as well. From operation 560 or operation 570 process 500 may also return to any of the aforementioned operations.

The exemplary embodiments of the invention illustrated and described in detail in the figures and foregoing description are illustrative and not limiting or restrictive. Only the presently preferred exemplary embodiments have been shown and described and all changes and modifications that come within the scope of the invention are to be protected. It should be understood that various features and aspects of the embodiments described above may not be necessary and embodiments lacking the same are also protected.

The following is a list of preferred embodiments of the invention:
1. A kit 100 comprising:
   a plurality of test elements 110 including a region 112 configured to receive a sample of fluid and a reagent adapted to react with the sample of fluid; and
   a memory unit 120 having a first set of memory locations 122 and a second set of memory locations 122, the first set of memory locations 122 storing encrypted calibration information relating to the plurality of test elements, and the second set of memory locations 122 being password protected.
2. A kit 100 according to embodiment 1 wherein the memory unit 120 includes a third set of memory locations 122 storing audio content, gaming content, video content, visual content, or a combination thereof.
3. A kit 100 according to embodiment 1 wherein the test elements 110 are blood glucose test strips and the reagent is operable to react with the sample to provide information indicative of blood glucose concentration.
4. A kit 100 according to embodiment 1 wherein the memory unit 120 includes flash memory.
5. A kit 100 according to embodiment 1 wherein the test elements 110 and the memory unit 120 are provided in common packaging 130.
6. A kit 100 according to embodiment 1 further comprising a meter 300 operable to communicate with the test elements 110 and the memory unit 120.
7. A kit according to embodiment 6 wherein the first set of memory locations 122 stores encrypted computer code and the meter 300 is operable to decrypt the calibration information, decrypt the computer code, and analyze the sample using the decrypted calibration information and the decrypted computer code.
8. A kit 100 according to embodiment 6 wherein the meter 300 is operable to store password protected information of a measurement of the sample in the second set of memory locations 122.
9. A kit 100 according to embodiment 1 wherein the first set of memory locations 122 includes hidden memory locations 122.
10. A kit 100 according to embodiment 2 wherein the first set of memory locations 122, the second set of memory locations 122 and the third set of memory locations 122 are partitions of a common memory.
11. An apparatus comprising:
   a memory unit 120 including first memory locations 122 storing calibration information for a set of test elements 110, the calibration information being encrypted, second password protected memory locations 122; and third memory locations 122 storing audio content, gaming content, video content, visual content, or a combination thereof.
12. An apparatus according to embodiment 11 further comprising the set of test elements 110.
13. An apparatus according to embodiment 9 further comprising a meter 300 operable to communicate with the memory unit 120 and the test elements 110.
14. An apparatus according to embodiment 13 wherein the meter is operable to communicate with the memory unit, decrypt the calibration information, and analyze a sample provided to the test element using the decrypted calibration information.
15. An apparatus according to embodiment 13 wherein the meter 300 is operable to communicate with the memory unit 120 to obtain decrypted calibration information and to analyze a sample provided to the test element 110 using the decrypted calibration information.
16. An apparatus according to embodiment 15 wherein the meter 300 is operable to store information of the analysis of the sample in the second password protected memory locations 122; wherein access to the information of the analysis is controlled based upon a password.
17. An apparatus according to embodiment 13 wherein the first memory locations 122 store encrypted software and the meter 300 is operable to communicate with the memory unit 120 to obtain decrypted calibration information and decrypted software and to analyze a sample provided to the test element 110 using the decrypted calibration information and the decrypted software.
18. An apparatus according to embodiment 17 wherein the meter 300 is operable to store password-protected information of the analysis in the second password protected memory locations.
19. A method comprising:
   providing a test element 110, a memory unit 120, and a meter 300, the test element 110 including a reagent and being adapted to receive a sample of fluid and to react the sample of fluid with the reagent, the memory unit 120 including a first set of memory locations 122 storing encrypted calibration information relating to the test element, and a second set of memory locations 122 being password protected, the meter 300 operable to communicate with the test element 110 and the memory unit 120;
   establishing communication between the memory unit 120 and the meter 300 effective to provide decrypted calibration information at the meter 300;
   receiving the sample of fluid at the test element 110 and reacting the sample of fluid with the reagent;
   establishing communication between the test element 110 and the meter 300; and
   measuring an analyte in the sample of fluid using the meter 300, the test element 110 and the calibration information.
20. A method according to embodiment 19 further comprising storing information obtained from the measuring in the second set of memory locations 120; wherein the stored information is password protected.
21. A method according to embodiment 20 further comprising receiving a second sample of fluid at a second test element 110, establishing communication between the second test element 110 and the meter 300, measuring second information of an analyte using the meter 300, the second test element 110 and the calibration information, and storing the second measured information in the second set of memory locations 122 wherein the second stored information is password protected.
22. A method according to embodiment 21 wherein the memory unit includes a third set of memory locations 122 storing audio content, gaming content, video content, visual content, or a combination thereof; the method further comprising controlling access to the audio content, the gaming content, the video content, the visual content, or the combination thereof based upon a characteristic of the information stored in the second set of memory locations 122.
23. A method according to embodiment 21 wherein the memory unit includes a third set of memory locations 122 storing audio content, gaming content, video content, visual content, or a combination thereof, the method further comprising allowing access to the audio content, the gaming content, the video content, the visual content, or the combination thereof based upon the presence of a predetermined number of stored information measurements.
24. A system comprising:
   a memory device 120 configured to store encrypted information in a first memory partition 122 and password protected information in a second memory partition 122; and
   a plurality of test elements 110 including a reagent and configured to receive a sample and to react the sample and the reagent, the plurality of test elements 110 sharing reactive characteristics amenable to common calibration;
   wherein the encrypted information includes common calibration information for the plurality of test elements.
25. A system according to embodiment 24 wherein the test elements 110 are electrochemical test elements and the calibration information includes information relating to a current response.
26. A system according to embodiment 24 wherein the memory device 120 is further configured to store media subject to digital rights management in a third memory partition 122.
27. A system according to embodiment 26 further comprising a testing device operable to communicate with the memory device to obtain at least a portion of the encrypted information in an intelligible form, operable to communicate with at least one of the test elements 110 to perform analysis of the sample received by the test element 110 and reacted with the reagent using the portion of the encrypted information in an intelligible form, and operable to store information of the analysis.
28. A system according to embodiment 27 wherein the information of the analysis is stored in the second memory partition 122 and is password protected.
29. A system according to embodiment 27 wherein the analysis of the sample includes electrochemical analysis.
30. A system according to embodiment 27 wherein the information of the electrochemical analysis is blood glucose concentration information.

## Claims

1. A kit (100) comprising:
a plurality of test elements (110) including a region (112) configured to receive a sample of fluid and a reagent adapted to react with the sample of fluid; and
a memory unit (120) having a first set of memory locations (122) and a second set of memory locations (122), the first set of memory locations (122) storing calibration information relating to the plurality of test elements, at least one of the first set of memory locations (122) and the second set of memory locations (122) being provided with a security feature;
wherein the memory unit (120) is insertable by a user into a meter (300) effective to provide the meter (300) access to the first set of memory locations (122) and the second set of memory locations (122).

2. The kit (100) according to claim 1 wherein the first set of memory locations (122) is configured to store encrypted calibration information relating to the plurality of test elements (110), and the second set of memory locations (122) is password protected.

3. The kit (100) according to claim 1 wherein the memory unit (120) includes a third set of memory locations (122) storing audio content, gaming content, video content, visual content, or a combination thereof.

4. The kit (100) according to claim 1 further comprising the meter (300) operable to communicate with the test elements (110) and the memory unit (120).

5. A kit (100) according to claim 4 wherein the calibration information is encrypted, the first set of memory locations (122) stores encrypted computer code and the meter (300) is operable to decrypt the calibration information, decrypt the computer code, and analyze the sample using the decrypted calibration information and the decrypted computer code.

6. The kit (100) according to claim 4 wherein the meter (300) is operable to store password protected information of a measurement of the sample in the second set of memory locations (122).

7. The kit (100) according to claim 1 wherein the first set of memory locations (122) includes hidden memory locations.

8. The kit (100) according to claim 3 wherein the first set of memory locations (122), the second set of memory locations (122) and the third set of memory locations (122) are partitions of a common memory.

9. The kit (100) according to claim 1 wherein the security feature comprises encryption or password protection.

10. The kit according to claim 1 wherein the first set of memory locations (122) and the second set of memory locations (122) are provided with a security feature.

11. A kit (100) comprising:
a plurality of test elements (110) each being combinable with a meter (300) to measure a glucose concentration of a sample of biological fluid provided to a respective test element (110);
a memory unit (120) having a first set of memory locations (122) and a second set of memory locations (122), the first set of memory locations (122) storing calibration information relating to the plurality of test elements (110), the calibration information being protected with a security feature;
wherein the memory unit (120) is physically connectable to the meter (300) to provide the meter (300) access to the calibration information protected with a security feature.

12. The kit (100) according to claim 11 wherein the meter (300) is operable to store a plurality of glucose concentration measurements in the second set of memory locations (122), the plurality of glucose concentration measurements being protected with a second security feature.

13. The kit (100) according to claim 12 wherein the memory unit (120) includes a third set of memory locations (122) and the meter (300) and the memory unit (120) are operable to control user access to the third set of memory locations (122) based upon a number of glucose concentration measurements in the second set of memory locations (122).

14. A kit according to claim 11 wherein the calibration information relating to the plurality of test elements stored the first set of memory locations (122) is encrypted.

15. The kit (100) according to claim 12 wherein the plurality of glucose concentration measurements stored the second set of memory locations (122) are encrypted.
